(19) Europäisches Patentamt

European Patent Office

Office européen des brevets

(11) Veröffentlichungsnummer: **0 474 200 B1**

(12) **EUROPÄISCHE PATENTSCHRIFT**

(45) Veröffentlichungstag der Patentschrift: **14.12.94**

(51) Int. Cl.5: **C07C 51/487**

(21) Anmeldenummer: **91114867.4**

(22) Anmeldetag: **03.09.91**

(54) **Verfahren zur Racematspaltung von 2,2-Dimethylcyclopropancarbonsäure.**

(30) Priorität: **04.09.90 CH 2866/90**

(43) Veröffentlichungstag der Anmeldung:
**11.03.92 Patentblatt 92/11**

(45) Bekanntmachung des Hinweises auf die
Patenterteilung:
**14.12.94 Patentblatt 94/50**

(84) Benannte Vertragsstaaten:
**AT BE CH DE DK ES FR GB IT LI NL SE**

(56) Entgegenhaltungen:
**GB-A- 1 596 033**
**US-A- 2 388 688**

**PATENT ABSTRACTS OF JAPAN, vol. 7, no.
122, (C-168)(1267), 26. März 1983; JP-A-58 041
847**

(73) Patentinhaber: **LONZA AG**

**CH-3945 Gampel/Wallis (CH)**

(72) Erfinder: **Meul, Thomas, Dr.**
**Meisenweg 1**
**Visp (Kanton Wallis) (CH)**

(74) Vertreter: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

## Beschreibung

Gegenstand der vorliegenden Erfindung ist ein Verfahren zur Herstellung optisch reiner 2,2-Dimethylcyclopropancarbonsäure durch Racematspaltung.

Das Amid der 2,2-Dimethylcyclopropancarbonsäure ist ein wichtiges Zwischenprodukt für die Synthese des Enzyminhibitors Cilastatin (EP 0 048 301).

Insbesondere für die Herstellung von pharmazeutischen Wirkstoffen ist es wünschenswert, 2,2-Dimethylcyclopropancarbonsäure in optisch reiner Form, d.h. in Form des reinen (S)-(+)- oder des reinen (R)-(-)-Enantiomeren zur Verfügung zu haben. Da die chemische Synthese vom 2,2-Dimethylcyclopropancarbonsäure die Verbindung in Form ihres Racemats liefert, ist es erforderlich, eine Spaltung dieses Racemats durchzuführen. Solche Racematspaltungen werden üblicherweise dadurch bewirkt, dass man zunächst mittels einer optisch aktiven Hilfssubstanz das zu trennende Enantiomerengemisch in ein Gemisch diastereomerer Derivate überführt, welches sich aufgrund der unterschiedlichen physikalischen Eigenschaften von Diastereomeren durch fraktionierende Kristallisation oder Chromatographie trennen lässt. Aus den so getrennten Diastereomeren wird dann im Idealfall jeweils ein reines Enantiomeres der zu trennenden Verbindung und die optisch aktive Hilfssubstanz in Freiheit gesetzt.

In der Realität gelingt mit einer gegebenen Hilfssubstanz, auch wenn diese optisch völlig rein ist, meistens nur die unvollständige Abtrennung eines reinen Enantiomeren, so dass ein Gemisch zurückbleibt, welches überwiegend aus dem anderen Enantiomeren besteht. In weniger günstigen Fällen kann keines der beiden Enantiomeren in reiner Form isoliert werden.

Als Derivate von Carbonsäuren zu Zweck der Racematspaltung werden häufig deren Salze mit optisch aktiven Basen, insbesondere Aminen, verwendet. Diese Salze haben den Vorteil, dass sie sich sehr leicht und schnell bilden und durch Zusatz einer starken Säure auch wieder spalten lassen. Zur Racematspaltung von 2,2-Dimethylcyclopropancarbonsäure wurden bereits (S)-(-)-1-Phenylethylamin (GB-PS 1 260 847), (-)-N-Methylephedrin (JP-Anm. 60 56 936 und 60 56 942), Chinin (EP-Anm. 0 161 546) und verschiedene 1,2-Diphenylethylamine (EP-Anm. 0 093 511) eingesetzt.

Mit 1-Phenylethylamin konnte weder eine befriedigende Ausbeute noch eine ausreichende optische Reinheit erreicht werden. Chinin lieferte ein Enantiomeres in guter optischer Reinheit, aber schlechter Ausbeute, für N-Methylephedrin wurde keine Ausbeute angegeben. Bei 1,2-Diphenylethylamin ist die Ausbeute befriedigend und die optische Reinheit sehr gut, das Reagens ist jedoch, wie auch N-Methylephedrin, sehr teuer.

Weiterhin ist bekannt, dass 2,2-Dimethylcyclopropancarbonsäure über die diastereomeren Menthylester, welche aus dem Säurechlorid mit (+)- bzw. (-)-Menthol erhältlich sind, in die Enantiomeren getrennt werden kann (US-PS 4 487 956). Dieses Verfahren liefert zwar brauchbare Ausbeuten und optische Reinheiten, ist jedoch relativ umständlich bei der Aufarbeitung und benötigt das relativ teure Menthol. EP-A-0 461 541 beschreibt ein Verfahren zur Racematspaltung von 2,2-Dimethylcyclopropancarbonsäure durch Veresterung mit Mandelsäuremethylester.

Aufgabe der vorliegenden Erfindung war daher, ein Verfahren zur Racematspaltung von 2,2-Dimethylcyclopropancarbonsäure zur Verfügung zu stellen, welches einfach durchzuführen ist und mit preiswerten optisch aktiven Hilfssubstanzen beide Enantiomeren in guter Ausbeute und hoher optischer Reinheit zu gewinnen erlaubt.

Erfindungsgemäss wird diese Aufgabe durch das Verfahren nach Patentanspruch 1 gelöst.

Es wurde gefunden, dass optisch aktives 1-(3-Methoxyphenyl)ethylamin mit racemischer 2,2-Dimethylcyclopropancarbonsäure diastereomere Salze bildet, die sich in ihrer Löslichkeit wesentlich unterscheiden, so dass bereits durch einmalige Umkristallisation das schwerer lösliche Diastereomer weitgehend rein und in guter Ausbeute erhalten werden kann.

Optisch aktives 1-(3-Methoxyphenyl)ethylamin kann durch Racematspaltung von (±)-1-(3-Methoxyphenyl)ethylamin mit optisch aktiven Säuren, z.B. Aepfelsäure (JP-Anm. 58 041 847, C.A. 99 194949b) hergestellt werden. (±)-1-(3-Methoxyphenyl)ethylamin ist aus 3-Methoxyacetophenon nach bekannten Verfahren herstellbar (E. Schlittler und J. Müller, Helv.Chim.Acta 31, 914-924 (1948)).

Die Salzbildung der racemischen 2,2-Dimethylcyclopropancarbonsäure mit dem optisch aktiven 1-(3-Methoxyphenyl)ethylamin wird vorteilhaft in einem geeigneten Lösungsmittel durchgeführt. Geeignete Lösungsmittel sind alle Lösungsmittel, in welchen die beiden Substanzen hinreichend löslich sind und die nicht selbst mit einer der beiden Substanzen eine Reaktion eingehen, also Wasser und alle neutralen organischen Lösungsmittel sowie deren Gemische. Vorzugsweise werden solche Lösungsmittel verwendet, in denen auch die entstehenden Salze eine gewisse Löslichkeit besitzen, so dass es möglich ist, letztere daraus auch umzukristallisieren. Besonders bevorzugt ist Wasser allein oder im Gemisch mit bis zu 25 Vol% eines oder mehrerer Alkohole mit 1 bis 3 C-Atomen, also Methanol, Ethanol, 1-Propanol oder 2-Propanol, insbesondere Methanol.

Wie bei Salzbildungen üblich, ist die Reaktionstemperatur nicht kritisch, vorzugsweise wird die Reaktion daher etwa bei Raumtemperatur durchgeführt. Bei Verwendung der bevorzugten Wasser/Alkohol-Gemische als Reaktionsmedium wird die Lösungsmittelmenge vorzugsweise so gewählt, dass beim Abkühlen des Reaktionsgemisches auf beispielsweise ca. 0°C im wesentlichen nur das schwerer lösliche Diastereomer auskristallisiert. Hierbei handelt es sich um das Diastereomer, in welchem die asymmetrischen C-Atome von Säure und Amin entgegengesetzte Konfiguration aufweisen, also bei Verwendung von (R)-(+)-1-(3-Methoxyphenyl)-ethylamin das Salz mit (S)-(+)-2,2-Dimethylcyclopropancarbonsäure und bei Verwendung des (S)-(-)-Amins das Salz mit der (R)-(-)-Säure. Es genügt dann in der Regel eine Umkristallisation, wobei vorzugsweise das gleiche Lösungsmittel wie für die Salzbildung verwendet wird, um ein Produkt mit einer optischen Reinheit von ca. 95% zu erhalten.

Aus dem so erhaltenen diastereomeren Salz wird durch Zusatz einer starken Säure das entsprechende Enantiomer der 2,2-Dimethylcyclopropancarbonsäure freigesetzt. Als starke Säure wird vorzugsweise Salzsäure oder (wässrige) Schwefelsäure verwendet.

Dieser Schritt wird vorzugsweise in Wasser durchgeführt, wobei die Wassermenge zweckmässig so gewählt wird, dass das entstehende Salz der starken Säure mit dem Amin vollständig gelöst wird.

Die Hauptmenge der optisch aktiven 2,2-Dimethylcyclopropancarbonsäure scheidet sich dann als Öl ab,und der in der wässrigen Phase gelöste Teil kann durch Extraktion mit einem unpolaren Lösungsmittel gewonnen werden. Als unpolares Lösungsmittel wird vorzugsweise ein Alkan mit 5 bis 8 C-Atomen, also beispielsweise Pentan, Hexan, Heptan, Octan, Isooctan, Cyclohexan oder Methylcyclohexan verwendet, das durch Destillation von der 2,2-Dimethylcyclopropancarbonsäure leicht abgetrennt werden kann. Besonders bevorzugt ist (n-)Hexan. Die so erhaltene optisch aktive 2,2-Dimethylcyclopropancarbonsäure kann gegebenenfalls nach an sich bekannten Methoden in das entsprechende Amid übergeführt werden, beispielsweise durch Umsetzung des aus der Säure mit Thionylchlorid erhältlichen Säurechlorids mit Ammoniak.

Das in den Mutterlaugen der Kristallisationsschritte enthaltene Diastereomerengemisch wird vorteilhaft der gleichen Behandlung unterzogen, so dass daraus ein Gemisch der beiden Enantiomeren der 2,2-Dimethylcyclopropancarbonsäure erhalten wird, in welchem das mit dem kristallinen Salz abgetrennte Enantiomer stark abgereichert ist. Dieses Enantiomerengemisch kann nun entweder zur Gewinnung des reinen anderen Enantiomeren mit dem anderen Enantiomeren des 1-(3-Methoxyphenyl)ethylamins umgesetzt und damit dem erfindungsgemässen Verfahren unterzogen oder auf an sich bekannte Weise in ein Gemisch der enantiomeren Säurechloride übergeführt und durch Erhitzen auf 100-200°C vollständig racemisiert werden. Im letzteren Fall kann durch Hydrolyse des racemischen Säurechlorids wieder die racemische 2,2-Dimethylcyclopropancarbonsäure erhalten und dem erfindungsgemässen Verfahren zugeführt werden, so dass letztlich, von unvermeidbaren Verlusten abgesehen, die gesamte Menge des Racemats in ein reines Enantiomer umgewandelt werden kann.

Die optisch aktive Hilfssubstanz 1-(3-Methoxyphenyl)ethylamin schliesslich kann nach bekannten Methoden durch Zusatz einer starken Base zu der nach Abtrennung der 2,2-Dimethylcyclopropancarbonsäure zurückbleibenden Salzlösung und Extraktion zurückgenommen werden.

Die nachfolgenden Beispiele verdeutlichen die Durchführung des erfindungsgemässen Verfahrens.

Beispiel 1

(R)-1-(3-Methoxyphenyl)ethylammonium-(S)-2,2-dimethylcyclopropancarboxylat

In einem Gemisch von 750 ml Wasser und 7,5 ml Methanol wurden bei 55°C 75,9 g (R)-(+)-1-(3-Methoxyphenyl)ethylamin ($[\alpha]_D^{20} = +22,0$ (c=10, MeOH), opt. Reinheit (ee): 98,3%) und 57,3 g (±)-2,2-Dimethylcyclopropancarbonsäure gelöst und auf 0°C abgekühlt. Die ausgefallenen Kristalle wurden abfiltriert, getrocknet [Trockengewicht: 45,3 g,

$$[\alpha]_{365}^{20}: +101,6°$$

(c=3, Methanol)], bei 65°C in einem Gemisch aus 314 ml Wasser und 3,1 ml Methanol gelöst, durch Abkühlen auf 0°C wieder auskristallisiert, abfiltriert und getrocknet. Die Mutterlauge der Umkristallisation wurde beim nächsten Ansatz wieder als Lösungsmittel für die Salzbildung verwendet.
Ausbeute: 28,4 g
Schmp: 148-150°C

$$[\alpha]_{365}^{20}: +130,6°$$

(c=3, Methanol)

Beispiel 2

(S)-(+)-2,2-Dimethylcyclopropancarbonsäure

In 60 ml Wasser wurden 28,4 g (R)-1-(3-Methoxyphenyl)ethylammonium-(S)-2,2-dimethylcyclopropancarboxylat (aus Beispiel 1) aufgeschlämmt und mit 11,5 g 32%-iger Salzsäure versetzt.

Die (S)-(+)-2,2-Dimethylcyclopropancarbonsäure schied sich als Öl ab und wurde zweimal mit je 50 ml Hexan extrahiert.

Die Hexanphase wurde im Vakuum eingeengt und der Rückstand bei 95°C/20 Torr destilliert.

Ausbeute: 12,0 g (20,9%, bezogen auf das eingesetzte Racemat)

$[\alpha]_D^{20}$ : +140,4° (c = 1, CHCl$_3$)

Opt. Reinheit (GC): 93%

Beispiel 3:

Aufarbeitung der Mutterlauge und Racemisierung

Die in Beispiel 1 angefallene Mutterlauge der ersten Kristallisation (844,8 g) wurde mit 45,1 g 32%-iger Salzsäure versetzt und dreimal mit je 50 ml Hexan extrahiert.

Aus der Hexanphase erhielt man nach Abdestillieren des Lösungsmittels 39,4 g rohe (R)-(-)-2,2-Dimethylcyclopropancarbonsäure (Gehalt (GC): 90%, $[\alpha]_D^{20}$ = -51,8° (c = 1, CHCl$_3$) entspr. 39% opt. Reinheit) als farblose Flüssigkeit. Diese wurde mit 33,4 g Hexan verdünnt, auf 75°C erhitzt und innerhalb von 30 Minuten tropfenweise mit 55,5 g Thionylchlorid in 15,6 g Hexan versetzt. Nach 2,5 Stunden Rühren bei 75°C wurde das Hexan bei Normaldruck abdestilliert und der Rückstand 2 Stunden auf 135°C erhitzt. Anschliessend wurde das so erhaltene racemische Säurechlorid auf Raumtemperatur abgekühlt, zur Hydrolyse mit 140 g 20%-iger Natronlauge versetzt und eine Stunde auf 80°C erhitzt.

Die so erhaltene Salzlösung wurde auf Raumtemperatur abgekühlt, mit 41,1 g 32%-iger Salzsäure versetzt und dreimal mit je 50 ml Hexan extrahiert.

Nach Abdestillieren des Lösungsmittels und Vakuumdestillation des Rückstands erhielt man 29,6 g (83,5%, bezogen auf das eingesetzte Enantiomerengemisch) racemische 2,2-Dimethylcyclopropancarbonsäure als farblose Flüssigkeit.

Beispiel 4

Rückgewinnung des (R)-(+)-1-(3-Methoxyphenyl)-ethylamins

Die wässrigen Phasen aus Beispiel 2 (87,4 g) und Beispiel 3 (854,4 g) nach der Zersetzung der 1-(3-Methoxyphenyl)ethylammoniumsalze mit Salzsäure und Extraktion mit Hexan wurden vereinigt und mit 94g 25%-iger Natronlauge versetzt. Das reine Amin wurde dreimal mit je 100 ml Dichlormethan extrahiert.

Nach Abdestillieren des Losungsmittels und Vakuumdestillation des Rückstands erhielt man 62,1 g (R)-(+)-1-(3-Methoxyphenyl)ethylamin (Gehalt

(GC): 100%, $[\alpha]_D^{20}$ : +22,0° (c = 10, Methanol)) als farblose Flüssigkeit.

**Patentansprüche**

1. Verfahren zur Racematspaltung von 2,2-Dimethylcyclopropancarbonsäure durch Salzbildung mit einem optisch aktiven Amin, fraktionierende Kristallisation der gebildeten diastereomeren Salze, anschliessende Umsetzung der diastereomeren Salze mit einer starken Säure und Isolierung der freigesetzten optisch aktiven 2,2-Dimethylcyclopropancarbonsäure, dadurch gekennzeichnet, dass als optisch aktives Amin 1-(3-Methoxyphenyl)ethylamin eingesetzt wird.

2. Verfahren nach Patentanspruch 1, dadurch gekennzeichnet, dass die Salzbildung in Wasser mit einem Zusatz von 0-25 Vol.-% eines oder mehrerer Alkanole mit 1 bis 3 C-Atomen als Lösungsmittel durchgeführt wird.

3. Verfahren nach Patentanspruch 1 oder 2, dadurch gekennzeichnet, dass die fraktionierende Kristallisation mit Wasser mit einem Zusatz von 0-25 Vol.-% eines oder mehrerer Alkanole mit 1 bis 3 C-Atomen als Lösungsmittel durchgeführt wird.

4. Verfahren nach den Patentansprüchen 2 und 3, dadurch gekennzeichnet, dass die Salzbildung und die fraktionierende Kristallisation mit dem gleichen Lösungsmittel durchgeführt werden.

5. Verfahren nach einem oder mehreren der Patentansprüche 2 bis 4, dadurch gekennzeichnet, dass als Alkanol Methanol eingesetzt wird.

6. Verfahren nach einem oder mehreren der Patentansprüche 1 bis 5, dadurch gekennzeichnet, dass als starke Säure Salzsäure oder Schwefelsaure eingesetzt wird.

7. Verfahren nach einem oder mehreren der Patentansprüche 1 bis 6, dadurch gekennzeichnet, dass die Isolierung der optisch aktiven 2,2-Dimethylcyclopropancarbonsäure durch Extraktion mit einem unpolaren Lösungsmittel und anschliessende Destillation durchgeführt wird.

8. Verfahren nach Patentanspruch 7, dadurch gekennzeichnet, dass als unpolares Lösungsmittel ein Alkan mit 5 bis 8 C-Atomen, z.B. Hexan, eingesetzt wird.

9. Verfahren nach einem oder mehreren der Patentansprüche 1 bis 8, dadurch gekennzeichnet, dass das jeweils nicht verwendbare Enantiomere der 2,2-Dimethylcyclopropancarbonsäure durch Überführen in das Säurechlorid, Erhitzen auf 100 bis 200 °C und anschliessende Hydrolyse racemisiert und in das Verfahren zurückgeführt wird.

10. (R)-1-(3-Methoxyphenyl)ethylammonium-(S)-2,2-dimethylcyclopropancarboxylat.

11. (S)-1-(3-Methoxyphenyl)ethylammonium-(R)-2,2-dimethylcyclopropancarboxylat.

**Claims**

1. A process for the resolution of racemic 2,2-dimethylcyclopropane carboxylic acid by salt formation with an optically active amine, fractionating crystallization of the diastereomeric salts thus formed, subsequent reaction of the diastereomeric salts with a strong acid, and isolation of the thus released optically active 2,2-dimethylcyclopropane carboxylic acid, characterized by using 1-(3-methoxyphenyl) ethyl amine as an optically active amine.

2. The process according to Claim 1, characterized in that salt formation is conducted in water under addition of from 0 to 25% by volume of one or more alkanols having 1 to 3 carbon atoms as solvent(s).

3. The process according to Claim 1 or 2, characterized in that fractionating crystallization is conducted with water under addition of from 0 to 25% by volume of one or more alkanols having 1 to 3 carbon atoms as solvent(s).

4. The process according to Claims 2 and 3, characterized in that salt formation and fractionating crystallization are conducted with the same solvent.

5. The process according to one or more of Claims 2 to 4, characterized by using methanol as an alkanol.

6. The process according to one or more of Claims 1 to 5, characterized by using hydrochloric acid or sulfuric acid as a strong acid.

7. The process according to one or more of Claims 1 to 6, characterized in that isolation of the optically active 2,2-dimethylcyclopropane carboxylic acid is conducted by extraction with an unpolar solvent and subsequent distillation.

8. The process according to Claim 7, characterized by using as an unpolar solvent an alkane having 5 to 8 carbon atoms, e.g. hexane.

9. The process according to one or more of Claims 1 to 8, characterized in that the respective non-utilizable enantiomer of 2,2-dimethylcyclopropane carboxylic acid is racemized by conversion into the acid chloride, heating at 100 to 200 °C and subsequent hydrolysis, and is returned into the process.

10. (R)-1-(3-Methoxyphenyl) ethyl ammonium-(S)-2,2-dimethylcyclopropane carboxylate.

11. (S)-1-(3-Methoxyphenyl)-ethyl ammonium-(R)-2,2-dimethylcyclopropane carboxylate.

**Revendications**

1. Procédé pour le dédoublement ou dissociation racémique de l'acide 2,2-diméthylcyclopropanecarboxylique par salification avec une amine optiquement active, cristallisation fractionnante des sels diastéréo-isomériques formés, réaction consécutive des sels diastéréo-isomériques avec un acide fort et isolement de l'acide 2,2-diméthylcyclopropanecarboxylique actif optiquement libéré, caractérisé en ce qu'en tant qu'amine active optiquement on met en oeuvre une 1-(3-méthoxyphényl)éthylamine.

2. Procédé selon la revendication 1, caractérisé en ce que la salification est conduite dans l'eau avec une addition de 0,25% en volume d'un ou de plusieurs alkanols ayant 1 à 3 atomes C comme solvant.

3. Procédé selon la revendication 1 ou 2, caractérisé en ce que la cristallisation fractionnante avec l'eau est conduite avec une addition de 0,25% en volume d'un ou de plusieurs alkanols ayant 1 à 3 atomes C comme solvant.

4. Procédé selon les revendications 2 et 3, caractérisé en ce que la salification et la cristallisation fractionnante sont conduites avec le même solvant.

5. Procédé selon une ou plusieurs des revendications 2 à 4, caractérisé en ce qu'en tant qu'alkanol on met en oeuvre un méthanol.

6. Procédé selon une ou plusieurs des revendications précédentes 1 à 5, caractérisé en ce qu'en tant qu'acide fort en met en oeuvre de l'acide chlorhydrique ou de l'acide sulfurique.

7. Procédé selon une ou plusieurs des revendications 1 à 6, caractérisé en ce que l'isolement de l'acide 2-2-diméthylcyclopropanecarboxylique optiquement actif s'effectue par extraction avec un solvant apolaire et distillation consécutive.

8. Procédé selon la revendication 7, caractérisé en ce qu'en tant que solvant apolaire on met en oeuvre un alkane ayant 5 à 8 atomes C, par exemple hexane.

9. Procédé selon une ou plusieurs des revendications 1 à 8, caractérisé en ce que l'énantiomère respectivement non utilisable de l'acide 2-2-diméthylcyclopropanecarboxylique est racémisé par passage dans le chlorure d'acide, chauffage à 100 jusqu'à 200 °C, puis hydrolyse et recyclage dans le procédé.

10. (R)-1-(3-méthoxyphényl)éthylammonium-(S)-2,2-diméthylcyclopropanecarboxylique.

11. (S)-1-(3-méthoxyphényl)éthylammonium-(R)-2,2-diméthylcyclopropanecarboxylate.